# EUROPEAN PATENT APPLICATION

(11) **EP 4 825 968 A1**
(43) Date of publication of application: **21.10.2026**
(21) Application number: 25170371.6
(22) Date of filing: 14.04.2025
(51) Int. Cl.: C12N 15/113

(54) **A COMPOSITION FOR THE TARGETED INHIBITION OF THE EXPRESSION OF A GENE OF INTEREST IN A BIOLOGICAL CELL**

(71) Applicant: Eberhard Karls Universität Tübingen (Medizinische Fakultät), 72074 Tübingen (DE)
(72) Inventor: Skokowa, Julia, 72127 Kusterdingen (DE); Nasri, Masoud, 72074 Tübingen (DE); Dannenmann, Benjamin, 75387 Neubulach (DE); Findik, Betül, 72131 Ofterdingen (DE)
(74) Representative: Witte, Weller & Partner Patentanwälte mbB

(57) **Abstract**

The present invention relates to a composition for the targeted inhibition of the expression of a gene of interest in a biological cell, to a method for the targeted inhibition of the expression of a gene of interest in a biological cell involving said composition, a preparation comprising a biological cell prepared *in vitro* by said method, a kit for the targeted inhibition of the expression of a gene of interest in a biological cell, a method of treating a subject afflicted with a disease associated with the expression of a gene of interest by using said composition, and to a nucleic acid molecule intended for the targeted inhibition of the expression of a gene of interest in a biological cell.

## Description

### FIELD OF THE INVENTION

The present invention relates to a composition for the targeted inhibition of the expression of a gene of interest in a biological cell, to a method for the targeted inhibition of the expression of a gene of interest in a biological cell involving said composition, a preparation comprising a biological cell prepared *in vitro* by said method, a kit for the targeted inhibition of the expression of a gene of interest in a biological cell, a method of treating a subject afflicted with a disease associated with the expression of a gene of interest by using said composition, and to a nucleic acid molecule intended for the targeted inhibition of the expression of a gene of interest in a biological cell.

### BACKGROUND OF THE INVENTION

Congenital genetic disorders represent a diverse group of diseases caused by pathogenic variants in the human genome. Among these, a subset arises from *gain-of-function* mutations in non-essential genes, where the mutated gene product acquires novel or enhanced activity that disrupts normal cellular processes. Unlike loss-of-function mutations that typically result in gene inactivation, gain-of-function mutations often exert dominant effects and may lead to disease phenotypes even in the presence of a wild-type allele.

One illustrative example is severe congenital neutropenia (CN), also often known as Kostmann syndrome or disease, an inherited pre-leukemia bone marrow failure syndrome. CN manifests in infancy with life-threatening bacterial infections. It causes severe pyogenic infections. CN is caused in about half of cases by autosomal dominant *ELANE* mutations (*ELANE*-CN), encoding neutrophil elastase (NE) protein. CN patients suffer from severe, life-threatening bacterial infections starting early after birth due to the absence or very low numbers of neutrophils in peripheral blood.

Daily injection of recombinant human granulocyte colony-stimulating factor (rhG-CSF) is the standard treatment option for CN patients. However, not all patients respond to rhG-CSF and approximately 15 % of ELANE-CN patients develop myelodys-plastic syndrome (MDS) or acute myeloid leukemia (AML). Hematopoietic stem cell transplantation (HSCT) is the ultimate curative option for CN patients, but it carries risks of adverse events and possible mortality.

Disease phenotype correction by gene editing of patients' CD34⁺ hematopoietic stem and progenitor cells (HSPCs) *ex vivo,* followed by autologous transplantation, may offer an alternative curative therapy.

It has been reported that knocking out *ELANE* is a feasible universal gene editing strategy for *ELANE*-CN, regardless of the mutation position; cf. Nasri et al., Haematologica, 2020, Vol. 105(3):598-609.

In the WO 2022/175162 a CRISPR/Cas9 based gene editing method is disclosed targeting the promoter of the *ELANE* gene instead of the coding sequence, which is suggested for the treatment of CN.

However, even some of the known gene editing methods for the treatment of hereditary diseases represent promising approaches with reasonable off-target profiles, *e.g*., as described in WO 2022/175162, there remains a need for further improvements in terms of safety, efficacy, and applicability. In particular, the intervention in the integrity of the DNA entails safety risks that cannot yet be fully understood in their entirety and long-term effects. It is therefore the purpose of the present invention to provide treatment options for hereditary diseases that can be used to prevent or at least reduce the disadvantages of the prior art. In particular, tools for a gene editing method are to be provided that address the safety concerns of the known gene editing methods and therefore enables an improved safety profile.

The present invention fulfills these and other needs.

### SUMMARY OF THE INVENTION

The present invention provides a composition for the targeted inhibition of the expression of a gene of interest in a biological cell, comprising:
- a protein comprising adenine deaminase activity or a nucleic acid molecule encoding said protein, and a
- a single guide RNA (sgRNA) configured to hybridize to a TATA box of a promoter controlling the expression of the gene of interest.

According to the invention, a "composition" refers to any combination of two or more components, which may be chemical, biological, or biochemical in nature. The components may be present in any physical state and may include, without limitation, proteins, peptides, nucleic acids, vectors, buffers, excipients, delivery vehicles, or any mixtures thereof, formulated for a specific biological, therapeutic, diagnostic, or research purpose.

A "gene of interest" is any nucleotide sequence within or derived from a genome that is selected for modulation, such as inhibition. The gene may encode a protein and may be endogenous or exogenous to the biological system under study. The selection of the gene is context-dependent and may be based on its biological function, disease association, or utility in experimental or therapeutic applications.

"Targeted inhibition of expression" refers to a specific, directed reduction or complete suppression of the transcription and/or translation of a particular gene, such as the gene of interest. This inhibition is achieved through molecular tools or interventions that recognize and act upon a defined regulatory element, such that gene expression is reduced without broadly affecting unrelated genes or cellular pathways.

"Adenine deaminase activity" is the biochemical capability of an enzyme or protein complex to catalyze the deamination of adenine bases in nucleic acids, particularly converting adenine (A) to inosine (I), which is interpreted as guanine (G) during replication or transcription. This activity may occur in the context of DNA or RNA substrates and may be intrinsic to the protein or conferred via fusion with other nucleic acid-targeting domains such as programmable nucleases. The activity can be embodied by the enzyme itself, i.e. an adenine deaminase either as a standalone protein or as a fusion protein or an adenine deaminase domain of a fusion protein.

The "TATA box of a promoter" refers to a conserved DNA sequence, typically comprising the nucleotides TATAAA or a close variant, located upstream of the transcription start site of many eukaryotic genes, such as the gene of interest according to the invention. It serves as a core promoter element recognized by the transcriptional machinery, including TATA-binding protein (TBP) and associated factors, and is critical for the initiation of gene transcription.

A "single guide RNA" (sgRNA) is a synthetic RNA molecule designed to direct an associated enzyme, such as an editing enzyme or a nuclease, to a specific nucleic acid target sequence, such as a gene or a regulatory element thereof. It comprises a guide sequence that is complementary or essentially complementary to the target and a scaffold region that facilitates binding to the effector protein. The sgRNA confers sequence specificity and is a key component in CRISPR-based editing systems. According to the invention the sgRNA's sequence is designed to be complementary or essentially complementary to a DNA region encompassing or overlapping the TATA box motif within the promoter of the gene of interest. The "hybridization", therefore, means a specific binding under stringent conditions to the TATA box. The sgRNA guides the protein comprising adenine deaminase activity to the specific genomic locus by base-pairing with the DNA strand that contains the TATA sequence. Hybridization of the sgRNA to the TATA box ensures localized activity of the adenine deaminase, allowing the targeted deamination of adenine residues within the TATA motif, e.g., converting TATA to TGTG, thereby impairing transcription initiation and leading to reduced or abolished expression of the downstream gene of interest.

The objective underlying the invention is thus fully achieved. It is particularly advantageous that, unlike existing strategies, *e.g*., described in WO 2022/175162 (*loc. cit*.), there are no DNA strand breaks. Furthermore, the coding region of the gene of interest remains unaffected. The composition according to the invention therefore enables targeted expression control with minimal interference with the integrity of the genome, thereby maximizing safety. The composition according to the invention is therefore particularly suitable for therapeutic use in humans, especially for the treatment of (mono-)genetically caused diseases, especially those involving non-essential genes.

In a preferred embodiment of the composition according to the invention the protein comprising adenine deaminase activity is a CRISPR/Cas9 adenine base editor (ABE).

A "CRISPR/Cas9 adenine base editor" (ABE) is a genome editing tool composed of a catalytically impaired or "nicking" variant of the CRISPR-associated protein 9 (Cas9) fused to an evolved adenine deaminase enzyme. The catalytically impaired Cas9 is a modified form of Cas9 in which one or both of these nuclease domains have been mutated to inactivate their cutting function. Depending on which domains are mutated, two main types of impaired Cas9 are commonly used. One is nickase Cas9 (nCas9), where only one of the two nuclease domains (usually HNH or RuvC) is active. This means it introduces a single-strand break (called a "nick") instead of a double-strand break. The other is dead Cas9 (dCas9), where both nuclease domains are mutated (e.g., D10A + H840A), so the Cas9 binds to DNA at the target site but does not cut either strand. It acts as a DNA-binding platform to recruit other enzymes (e.g., transcriptional re-pressors, epigenetic modifiers, or base editors). An evolved adenine deaminase enzyme refers to a genetically engineered version of a natural deaminase enzyme that has been modified through protein engineering techniques to perform adenine-to-inosine (A→I) conversion in DNA, rather than in its native context (which is usually RNA or free adenine).

The ABE complex is guided to the gene's TATA box by the specifically configured sgRNA, where it mediates the targeted deamination of adenine (A) to inosine (I), which is read as guanine (G) by cellular DNA replication and repair machinery, resulting in the conversion of TATA to TGTG. The system enables precise, programmable A•T to G•C base pair conversions in double-stranded DNA without inducing double-strand breaks. ABEs can function in a wide range of prokaryotic and eukaryotic cells and are commonly engineered for enhanced activity, fidelity, and sequence compatibility, including but not limited to ABE7.10, ABE8e, and ABE8.20-m variants.

In this embodiment, the ABE can also be used as a component of a 'ribonucleoprotein' (RNP) complex, i.e. the combination of Cas protein (e.g. nCas9) and the sgRNA according to the invention. This means that the fully assembled Cas protein and guide RNA are introduced directly into cells - without the administration of DNA or mRNA, from which these components would first have to be synthesized in the body. This ensures a rapid onset of action.

This embodiment, therefore, leverages a highly targeted genome-editing system capable of converting specific adenine (A) bases to guanine (G) without inducing double-strand breaks (DSBs). This minimizes off-target genomic damage and enhances cell viability.

In a further embodiment of the composition according to the invention said gene of interest is a non-essential gene.

As used herein, the term "non-essential gene" refers to a gene whose expression is not required for cell viability or basic cellular functions, such that targeted inhibition of its expression does not or essentially not result in deleterious effects to the cell. In certain embodiments, the non-essential gene is causally linked to a disease state due to overexpression or gain-of-function mutation, and its transcriptional downregulation via TATA box editing serves as a therapeutic intervention.

By specifically targeting non-essential genes, the approach reduces the risk of adverse cellular effects resulting from gene silencing. The loss of expression does not compromise cell viability or function, making the system safer for both *ex vivo* and *in vivo* therapeutic applications. This embodiment is particularly valuable in diseases where overexpression or aberrant expression of a non-essential gene is causative or pathogenic for example, in dominant gain-of-function mutations. Silencing such genes can alleviate disease symptoms without risking essential cellular functions. Instead of deleting the gene, the system alters the promoter, e.g., the TATA box, leading to sustained transcriptional repression without changing coding sequences or introducing double-strand breaks. This is especially advantageous in regulatory or therapeutic settings. This embodiment supports applications in diverse genetic disorders - including congenital diseases, cancers, and developmental disorders - where selective gene downregulation is beneficial. Since the target genes are non-essential, the editing approach is more broadly applicable across different tissues, cell types, or patient populations, reducing the need for individualized risk-benefit assessments.

In a further embodiment of the invention the non-essential gene is associated with a congenital disease, preferably if present in mutated form, further preferably in a gain-of-function mutated form.

As used herein, the term "congenital disease" refers to a pathological condition present at birth that results from one or more genetic mutations. In preferred embodiments, the disease is associated with a mutation in a non-essential gene, wherein the mutation leads to increased, aberrant, or toxic activity of the encoded product (i.e., a gain-of-function mutation). Such diseases may be amenable to treatment by reducing or abolishing expression of the mutated gene via targeted editing of its promoter region, for example by disrupting the TATA box sequence.

Instead of attempting to correct the mutated coding sequence, which is technically challenging and potentially risky, this embodiment proposes a transcriptional silencing strategy that bypasses the mutation by shutting down the gene's expression at the promoter level. Many congenital diseases caused by gain-of-function mutations follow an autosomal dominant inheritance pattern. In such cases, suppressing the expression of the mutant allele can be sufficient to alleviate or resolve the disease phenotype. Because the targeted gene is non-essential, its complete or partial transcriptional silencing is unlikely to lead to significant adverse effects, even when both alleles are suppressed. The approach is adaptable to a variety of congenital diseases in which disease etiology is tightly linked to aberrant expression of specific non-essential genes. This provides a platform technology for rare or monogenic disorders.

In yet another embodiment of the invention said congenital disease is selected from the group consisting of: congenital neutropenia (CN), cyclic neutropenia (CyN), familial hypercholesterolemia (FH), achondroplasia, Noonan syndrome, autosomal dominant forms of epilepsy, CAMK2-associated developmental disorders, androgen insensitivity.

"Congenital neutropenia" (CN) and "cyclic neutropenia" (CyN) belong to a group of inherited disorders characterized by a deficiency of neutrophils, leading to recurrent infections. Often caused by gain-of-function mutations in *ELANE,* a non-essential gene encoding neutrophil elastase.

"Familial hypercholesterolemia" (FH) is a genetic disorder marked by high LDL cholesterol levels due to mutations in *PCSK9* (among others). Gain-of-function mutations in *PCSK9* increase LDL receptor degradation, contributing to cardiovascular risk.

"Achondroplasia" is the most common cause of dwarfism, caused by a gain-of-function mutation in the *FGFR3* gene, which inhibits bone growth. *FGFR3* is often considered non-essential in the context of adult physiology.

The "Noonan Syndrome" describes a multisystem developmental disorder involving mutations in genes of the RAS/MAPK pathway (e.g., PTPN11, SOS1), often leading to dysregulated cell signaling via gain-of-function mechanisms.

"Autosomal dominant forms of epilepsy" include syndromes such as generalized epilepsy with febrile seizures plus (GEFS+), often linked to gain-of-function mutations in ion channel genes (SCN1A, KCNQ2, etc.) that alter neuronal excitability.

"CAMK2-associated developmental disorders" are caused by mutations in *CAMK2A* or *CAMK2B,* which encode subunits of Ca⁺⁺/calmodulin-dependent protein kinase II. Gain-of-function mutations can lead to neurodevelopmental deficits and seizures.

"Androgen insensitivity" is a disorder of sexual development caused by mutations in the *AR* gene (androgen receptor), often leading to resistance to androgens. Certain mutations increase receptor activity or alter signaling inappropriately.

The listed diseases are well-characterized monogenic disorders with established links to gain-of-function mutations in non-essential genes, making them ideal candidates for promoter-based silencing. Current treatments for these conditions are often symptomatic or limited in effectiveness. This embodiment offers a curative or disease-modifying approach by directly targeting the root cause - overexpression or hyperactivity of a mutant gene product. By choosing diseases where only the mutant allele or overactive gene needs to be silenced, the invention provides a precise, allele-agnostic strategy that avoids affecting unrelated cellular pathways. Since the targeted genes are non-essential (in many cases even in their wild-type form), transcriptional silencing via TATA box editing is unlikely to cause systemic toxicity or critical cellular dysfunction. The diseases listed span multiple organ systems - immune, skeletal, cardiovascular, auditory, and neurological - highlighting the versatility of the claimed composition and its potential as a platform technology.

In view of the above, in another embodiment of the invention the gene of interest is selected from the group consisting of: neutrophil elastase (*ELANE*), cochlin (*COCH*), proprotein convertase subtilisin/kexin type 9 (*PCSK9*), fibroblast growth factor receptor 3 (FGFR3), protein tyrosine phosphatase non-receptor type 11 (*PTPN11*), son of sevenless homolog 1 (*SOS1*), Ca⁺⁺/calmodulin-dependent protein kinase II (*CAMK2*), androgen receptor (*AR*).

This embodiment directly correlates specific gene targets to the congenital diseases listed in the pervious embodiment, strengthening the biological and therapeutic rationale of the invention. Each gene is known to contribute causally to a disease phenotype via overexpression or gain-of-function mutations. This embodiment provides clear molecular targets for the base editing system, facilitating the development of specific sgRNAs and editing protocols for each gene/promoter.

In another embodiment of the invention said biological cell is a hematopoietic cell, preferably a hematopoietic stem cell, further preferably primary human CD34⁺ hematopoietic stem cells, and highly preferably primary human CD34⁺ hematopoietic stem cells from patients with autosomal dominant gain-of-function mutations in *ELANE.*

Congenital diseases, particularly those caused by gain-of-function mutations, often result in systemic or tissue-specific dysfunctions that manifest early in life. When such diseases originate in the blood or immune system, hematopoietic cells - which give rise to all blood lineages - are a logical therapeutic target. Among hematopoietic cells, hematopoietic stem cells (HSCs) are of particular therapeutic interest because of their self-renewal capacity, multipotency, and well-established role in bone marrow transplantation protocols. CD34⁺ HSCs represent a clinically validated subset of human hematopoietic stem and progenitor cells. CD34⁺ cells are compatible with clinical-grade gene editing protocols, including CRISPR-based systems, and have a track record of safe reinfusion in patients. CD34⁺ hematopoietic stem cells from patients with autosomal dominant gain-of-function mutations in *ELANE* are of particular clinical interest. These mutations are causative in severe congenital neutropenia (CN) and cyclic neutropenia (CyN). CN and CyN are not only characterized by chronic neutrophil deficiency but are also pre-leukemic conditions, with a high lifetime risk of progression to acute myeloid leukemia (AML). The diseases originate in the hematopoietic stem cell compartment, making CD34⁺ cells the root source of both neutropenia and leukemogenic transformation. Therapeutically silencing *ELANE* in these cells, e.g., via TATA box editing, has the potential to correct neutrophil maturation and simultaneously prevent leukemic progression. This strategy avoids the limitations of symptomatic treatments like G-CSF and provides a mechanism-based, disease-modifying solution.

In another embodiment of the invention said sgRNA comprises a nucleotide sequence selected from the group consisting of: SEQ ID NO: 1 (sgRNA -2), SEQ ID NO: 2 (sgRNA -5), SEQ ID NO: 3 (sgRNA -29), and SEQ ID NO: 4 (sgRNA -81).

The sequences listed under SEQ ID NOs 1-4 correspond to single guide RNAs (sgRNAs) that are specifically designed to hybridize to the TATA box upstream of the *ELANE* gene. These sgRNAs direct the adenine deaminase activity or, e.g., the adenine base editor (ABE), to the TATA box region of the ELANE promoter, and enable precise A→G conversions within the core TATA motif (e.g., TATA → TGTG). This results in transcriptional silencing of the *ELANE* gene, whose overexpression or gain-of-function mutation is causative in congenital neutropenia.

Although the nucleotide sequences are listed in DNA format (i.e., using thymine, T), the invention expressly encompasses the corresponding RNA sequences, in which each thymine (T) is replaced by uracil (U). This reflects the biological reality that sgRNAs function in RNA form in CRISPR systems. However, sgRNAs are often synthesized as DNA templates for in vitro transcription, encoded in plasmid or viral vectors, or chemically synthesized directly as RNA. Thus, both DNA-based constructs encoding these guide sequences and the final RNA molecules themselves are included within the scope of the invention.

In another embodiment of the invention, the promoter comprises a nucleotide sequence of SEQ ID NO: 5.

This measure has the advantage of defining the exact region to be edited and supporting the design of complementary sgRNAs. SEQ ID NO: 5 encompasses the proximal promoter region of the *ELANE* gene, including the TATA box motif ("TATAA") which is a core regulatory element essential for transcription initiation. This region is the site of action for the complex of adenine deaminase activity or ABE and sgRNA of the invention. By targeting this specific sequence with a base editor, adenine residues in the TATA box (e.g., TATAA) can be precisely converted (e.g., TATA → TGTG), which disrupts promoter function, leading to suppressed transcription of *ELANE.*

In still another embodiment of the invention said nucleic acid molecule encoding said protein comprising adenine deaminase activity is mRNA.

In this embodiment the adenine deaminase activity - which may be part of a CRISPR adenine base editor (ABE) - is not delivered in its protein form, but rather as messenger RNA (mRNA) encoding the base editor enzyme. In particular, the mRNA may encode adenine deaminase. It may also encode an ABE fusion protein composed of an evolved adenine deaminase (e.g., TadA variant), fused to a Cas9 nickase or catalytically impaired Cas9.

This measure has the advantage of transient and controlled expression. mRNA allows for temporary production of the adenine deaminase activity or ABE in the target cell. This limits the duration of enzyme activity, thereby reducing off-target editing and immunogenicity - a key concern in clinical applications. The transient nature of mRNA expression enables precise titration of editing activity, and multiple doses can be administered if needed without long-term accumulation of the editing protein. Unlike DNA vectors (e.g., plasmids, viral constructs), mRNA does not integrate into the host genome, making it a safer alternative with lower risk of insertional mutagenesis. mRNA is directly translated in the cytoplasm, allowing faster onset of therapeutic activity compared to DNA-based delivery methods that require nuclear entry and transcription. mRNA can be efficiently delivered using lipid nanoparticles (LNPs) - a clinically validated system used in mRNA vaccines and emerging gene-editing therapies. This improves scalability and regulatory acceptance. Because only the RNA and not the protein is delivered exogenously, the immune system is less likely to react to foreign proteins introduced by transfection. ABEs are large fusion proteins and can be challenging to purify or express as recombinant protein complexes. Delivering them as mRNA simplifies formulation and avoids the complexity of protein-based delivery systems, especially in hard-to-transfect cells like primary human hematopoietic stem cells (CD34⁺ HSCs).

In another embodiment of the composition according to the invention, ABE is ABE8, preferably ABE8.20-m.

ABE8 refers to an advanced generation of adenine base editors that are derived from evolved forms of TadA deaminase, engineered for increased catalytic activity, broader editing windows, and higher editing efficiency. ABE8.20-m is a monomeric variant of ABE8. It combines a single-chain architecture (monomeric deaminase domain) with a Cas9 nickase, typically D10A mutant, resulting in a smaller, more compact, and efficient editing system.

ABE8 variants, including ABE8.20-m, display significantly enhanced A-to-G editing rates compared to earlier generations (e.g., ABE7.10), especially in primary cells and therapeutically relevant targets. These variants allow for base editing at a wider range of adenine positions within the target sequence, providing more flexibility in targeting critical adenines in the TATA box or other regulatory elements. ABE8.20-m has shown superior activity in primary human cells, including CD34⁺ hematopoietic stem cells, which are often resistant to efficient gene editing using earlier tools. The design of ABE8.20-m includes enhanced specificity, leading to fewer unintended edits, especially when paired with optimized sgRNAs - critical for therapeutic applications. The monomeric format of ABE8.20-m makes it easier to deliver, particularly when using size-constrained delivery vehicles like adeno-associated viruses (AAVs) or LNPs, which improves clinical feasibility. ABE8.20-m is compatible with both mRNA-based and protein-based (RNP) delivery strategies, offering flexibility for different therapeutic delivery platforms. ABE8.20-m represents a next-generation clinical-grade base editor, currently used in preclinical and early clinical studies, making it a credible and practical choice for real-world gene therapy applications.

Another subject-matter of the invention relates to a method for the targeted inhibition of the expression of a gene of interest in a biological cell, comprising the following steps:
1) providing a biological cell comprising a gene of interest controlled by a promoter comprising a TATA box,
2) introducing into said biological cell a composition comprising:
   - a protein comprising adenine deaminase activity or a nucleic acid molecule encoding said protein, and a
   - a single guide RNA (sgRNA) configured to hybridize to said TATA box of said promoter controlling the expression of said gene of interest,
3) incubating said cell and said composition, thereby allowing the binding of the protein comprising adenine deaminase activity to the promoter and converting adenine nucleotides in the TATA box to guanine nucleotides, preferably two adenine nucleotides to guanine nucleotides (TATA -> TGTG).

This embodiment translates the molecular concept into a concrete method, making the invention practically applicable in both research and therapeutic contexts. Preferably, said composition recited in steps (2) and (3) is the composition according to the invention.

The characteristics, features, advantages and further developments disclosed in relation with the composition of the invention also apply to the method according to the invention.

Another subject-matter of the invention relates to a preparation comprising a biological cell prepared *in vitro* by a method comprising the following steps:
1) providing a biological cell comprising a gene of interest controlled by a promoter comprising a TATA box,
2) introducing into said biological cell a composition comprising:
   - a protein comprising adenine deaminase activity or a nucleic acid molecule encoding said protein, and a
   - a single guide RNA (sgRNA) configured to hybridize to said TATA box of said promoter controlling the expression of said gene of interest,
3) incubating said cell and said composition, thereby allowing the binding of the protein comprising adenine deaminase activity to the promoter and converting adenine nucleotides in the TATA box to guanine nucleotides, preferably two adenine nucleotides to guanine nucleotides (TATA -> TGTG),
4) recovering said cell.

This cellular preparation is particularly suited for therapeutic administration to a human subject, such as a patient suffering from congenital neutropenia (CN). The cells can be hematopoietic stem or progenitor cells (HSPCs), preferably autologous CD34⁺ stem cells, which are collected from the patient, genetically modified *ex vivo* using the inventive method, and subsequently re-administered (reinfused) into the same patient. This approach enables a personalized, gene-targeted, long-lasting therapy that can potentially eliminate the disease phenotype by silencing aberrantly expressed genes such as *ELANE.*

The editing occurs *in vitro,* allowing for quality control, sequencing validation, and safety assessment of the edited cells before administration. The preferred use of the patient's own cells avoids immune rejection and minimizes ethical or regulatory barriers associated with allogeneic therapies. However, in an alternative embodiment the editing can also be carried out *in vivo.*

The preparation may be a pharmaceutical composition. Therefore, the preparation may comprise further components, if applicable, such as cryopreservation medium (*e.g*., DMSO, serum or serum-free additives), electrolyte-balanced infusion buffers (*e.g.,* Plasma-Lyte A, saline), stabilizers or osmoprotectants (*e.g.,* trehalose, mannitol), antibiotics or antifungals (for sterility during processing), growth factors (*e.g., SCF,* TPO, FLT3L) during *ex vivo* culture, carrier solutions (*e.g*., albumin-containing diluents), labeling agents (for cell tracking in clinical studies), etc.

The characteristics, features, advantages and further developments disclosed in relation to the preparation of the invention also apply to the method according to the invention.

Another subject-matter of the invention relates to a kit for the targeted inhibition of the expression of a gene of interest in a biological cell, comprising the composition according to the invention and instructions for delivering the composition to said biological cell so as to inhibit the expression of the gene.

Such a kit may be intended for use in research, *ex vivo* clinical gene editing, or therapeutic development workflows. The kit claim supports practical deployment of the invention, allowing for easy packaging, distribution, and standardized implementation in laboratories or clinical facilities. The kit may contain (i) the base editing protein (e.g., ABE8.20-m) or an mRNA/DNA construct encoding it, (ii) one or more sgRNAs (e.g., SEQ ID NOs 1-4), (iii) delivery tools (e.g., electroporation buffer, viral vector, lipid nanoparticles), (iv) optional controls and quality reagents, and (v) written instructions or protocols. Kits are well-suited for GMP-compatible workflows, making them attractive to biotech companies and hospitals working with gene therapy platforms. The inclusion of instructions for use promotes standardized application of the composition, improving safety, reproducibility, and efficacy. Therefore, such kits also allow semi-skilled laboratory staff to perform the method according to the invention.

The characteristics, features, advantages and further developments disclosed in relation to the preparation of the invention also apply to the kit according to the invention.

Another subject-matter of the invention relates to a nucleic acid molecule, preferably an RNA molecule, comprising a nucleotide sequence which is selected from the group consisting of: SEQ ID NO: 1 (sgRNA -2), SEQ ID NO: 2 (sgRNA -5), SEQ ID NO: 3 (sgRNA -29), and SEQ ID NO: 4 (sgRNA -81).

The listed sequences correspond to sgRNAs specifically designed to hybridize with the TATA box region of the ELANE gene promoter, enabling targeted A-to-G base editing at that site. This embodiment concerns the sequence-specific guides irrespective of how they are delivered or used, e.g., as standalone synthetic RNAs, as parts of plasmids, or within viral or non-viral vectors. Therefore, a "nucleic acid molecule" includes, without being limited to it, RNA molecules (*e.g*., chemically synthesized sgRNAs, in vitro transcribed sgRNAs), DNA templates (*e.g*., plasmid DNA, linear PCR products, gBlocks), or mRNA or vector-encoded sgRNA transcripts, including those used in viral vectors. The nucleic acid molecules can be used in therapeutic editing of patient-derived cells, such as CD34⁺ HSCs. They also be used in diagnostic assays, to assess promoter accessibility or editing efficiency, or in research tools, for validating promoter editing strategies in model systems.

The characteristics, features, advantages and further developments disclosed in relation with the preparation of the invention also apply to the nucleic acid molecule according to the invention.

Still another subject-matter of the invention relates to a method of treating a subject afflicted with a disease associated with a gene of interest, preferably a non-essential gene, further preferably a non-essential gene associated with a congenital disease, further preferably if present in mutated form, further preferably in a gain-of-function mutated form, comprising a step of administering to said subject the composition according or preparation according to the invention.

The features, characteristics, and embodiments of the composition according to the invention apply to this method according to the invention in a corresponding manner, even if not specifically indicated.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

The invention is now described and explained in further detail by referring to the following non-limiting examples and figures.

### BRIEF DESCRIPTION OF THE FIGURES

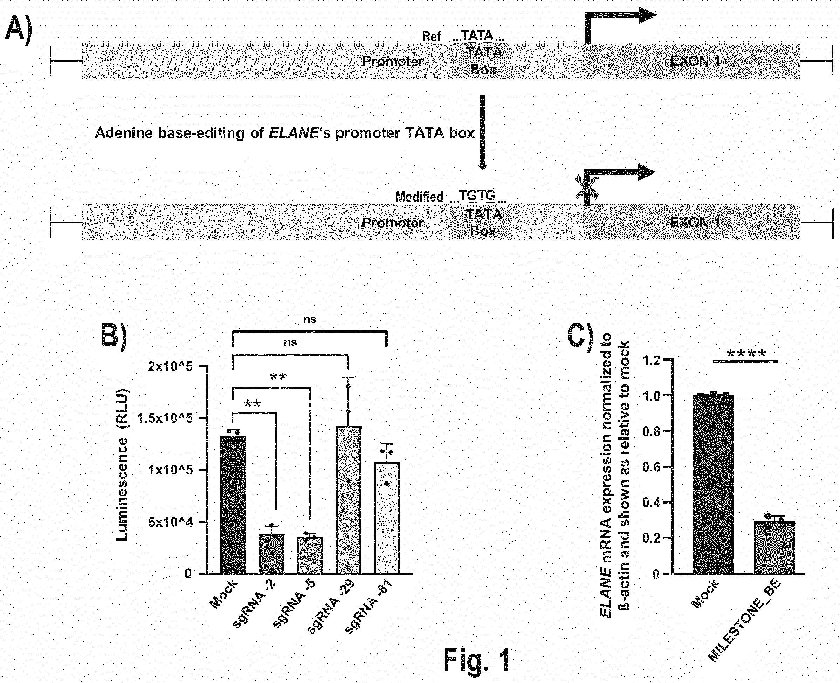

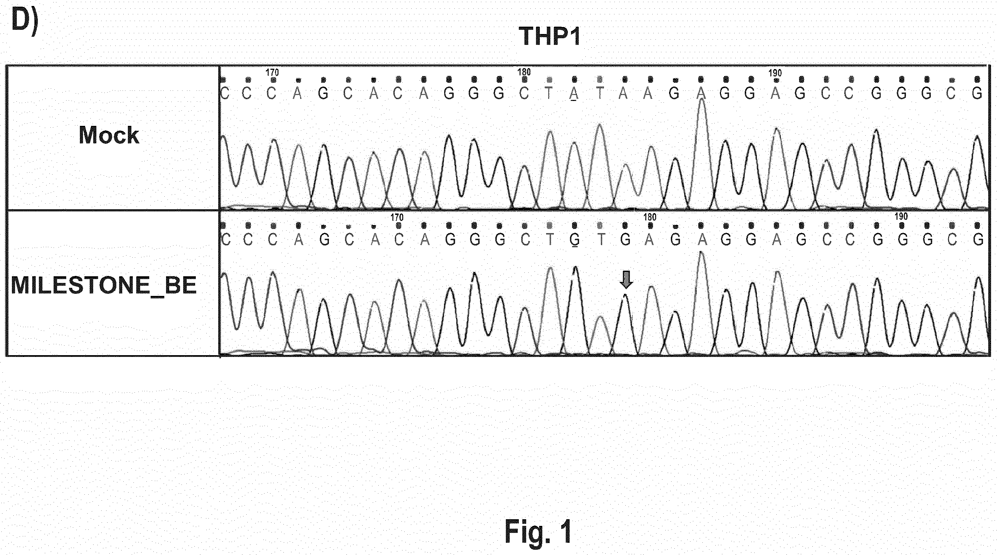

- Fig. 1: Establishment of a precise double-strand break-free strategy for reducing *ELANE* gene expression through adenine base editing of the *ELANE* gene promoter. A, Scheme of adenine base-editing strategy, targeting the *ELANE'*s promoter TATA box. B, Luminescence signal (RLU) of neutrophil elastase (NE)-HiBiT-tagged-THP-1 cells was measured 120 hours post-electroporation with different sgRNAs targeting -2, -5, -29 and -81 nucletoides upstream of the transcription start site in the *ELANE* promoter region using the GloMax system. Data represent means ± standard deviation (SD) from 3 independent experiments, One-way ANOVA with Dunnett's test, **P < 0.01, ns: not significant. C, *ELANE* mRNA expression in NE-HiBiT-tagged THP-1 cells was measured by qRT-PCR 14 days post-electroporation of the Mock or base-edited cells. *ELANE* mRNA expression was normalized to β-actin and is shown relative to the Mock group. Data represent means ± SD from three independent experiments. unpaired Student's t-test, ****P < 0.0001. D. Representative Sanger sequencing traces of DNA isolated from NE-HiBit-tagged THP-1 cells five days post-electroporation with the Mock or MILESTONE_BE editing, analyzed using ChromasPro software, are depicted.

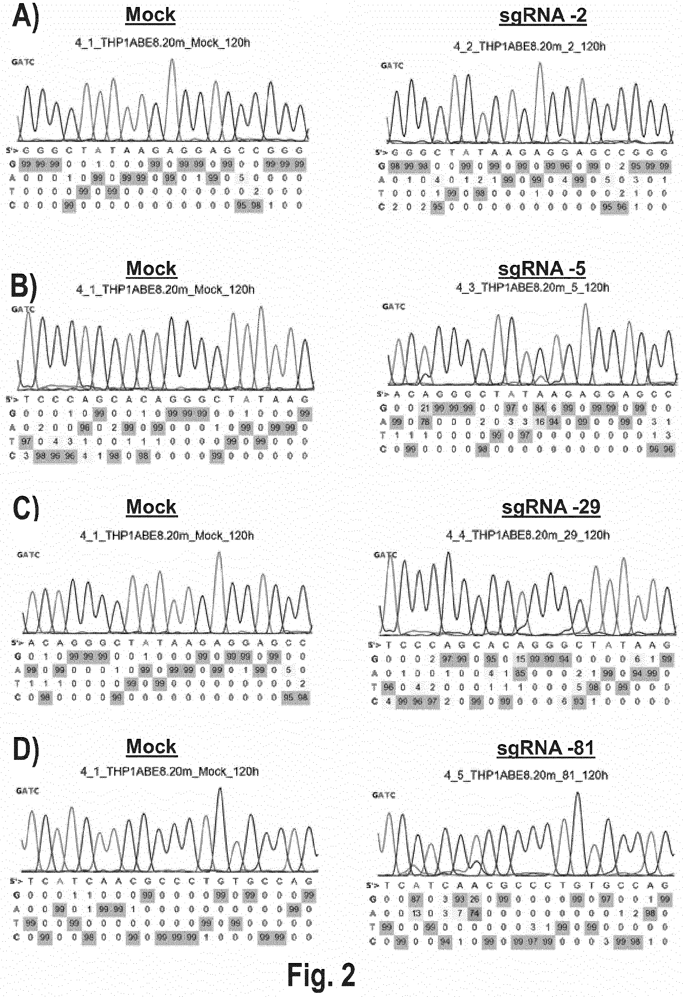

- Fig. 2: Representative Sanger sequencing traces of *ELANE*-promoter base-editing in THP-1 cells using base-editing analysis tool BEAT. A-D. Representative Sanger sequencing traces of DNA isolated from NE-HiBit-tagged THP-1 cells five days post-electroporation with Mock or MILESTONE_BE, analyzed using the online base editing analysis tool BEAT. The calculated percentage of each base at each position is depicted. Numbers in sgRNA title denotes the position on the *ELANE* core promoter upstream of transcription start site.

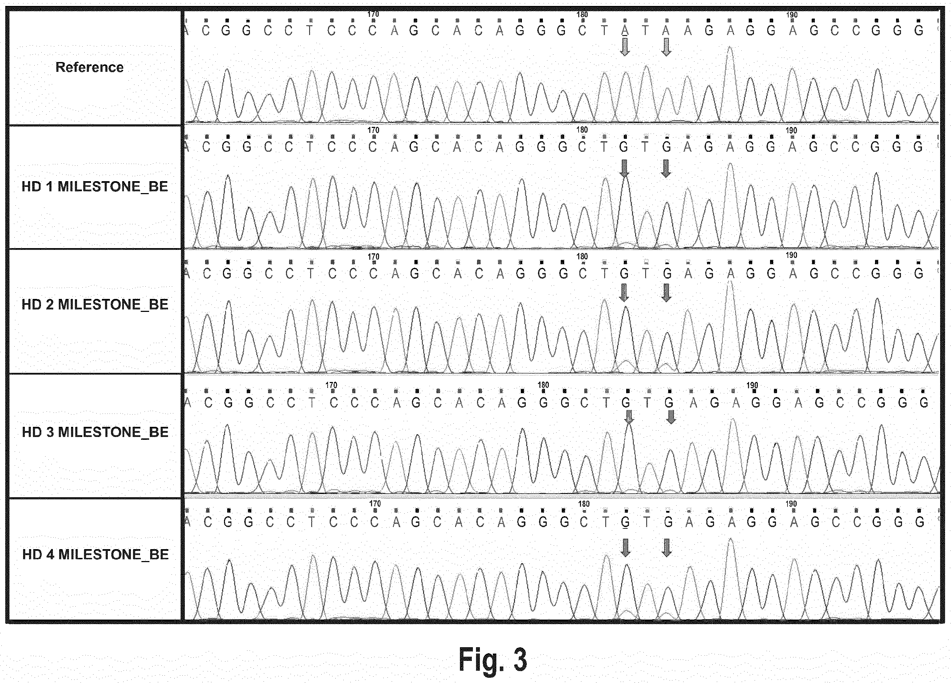

- Fig. 3: Representative Sanger sequencing traces of *ELANE*-promoter base-editing in four healthy donors using Chromas Pro software. Representative Sanger sequencing traces of DNA isolated from CD34⁺ cells 120 hours post-electroporation with Mock or MILESTONE_BE from four biological replicates. The arrows indicate the specific target adenine base editing sites within the DNA sequence. These sites are the intended locations where the base editor enzyme will attempt to modify the adenine nucleotide to a guanine nucleotide.

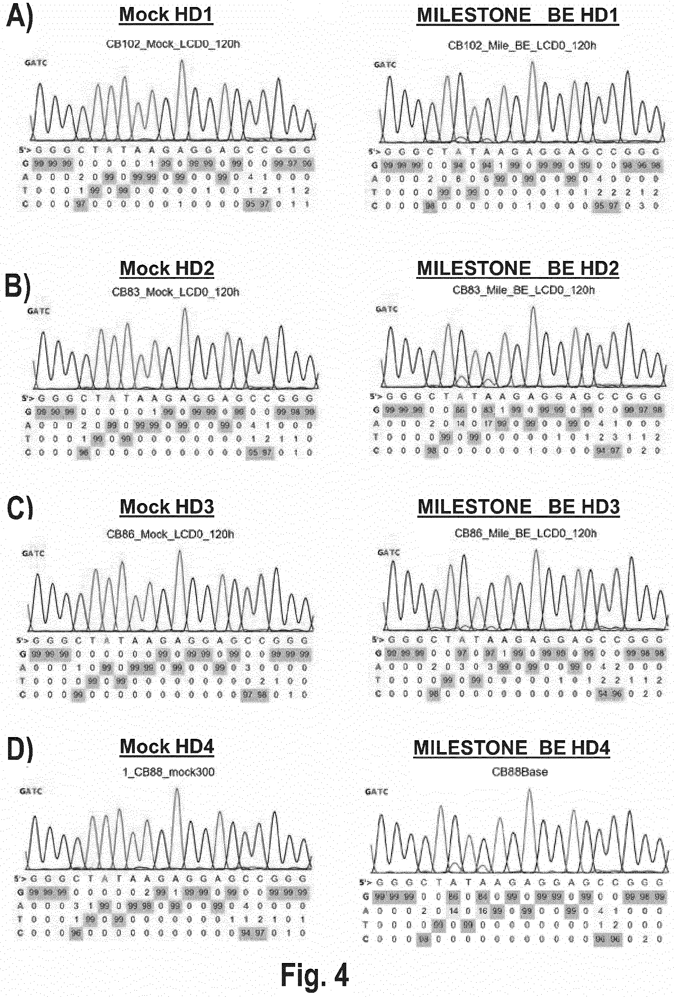

- Fig. 4: Representative Sanger sequencing traces of ELANE-promoter base-editing in four healthy donors using BEAT. A-D. Representative Sanger sequencing traces of DNA isolated from Mock and MILESTONE_BE CD34⁺ cells 120 hours post-electroporation from four biological replicates were analyzed using BEAT. The calculated percentage of each base at each position is depicted.

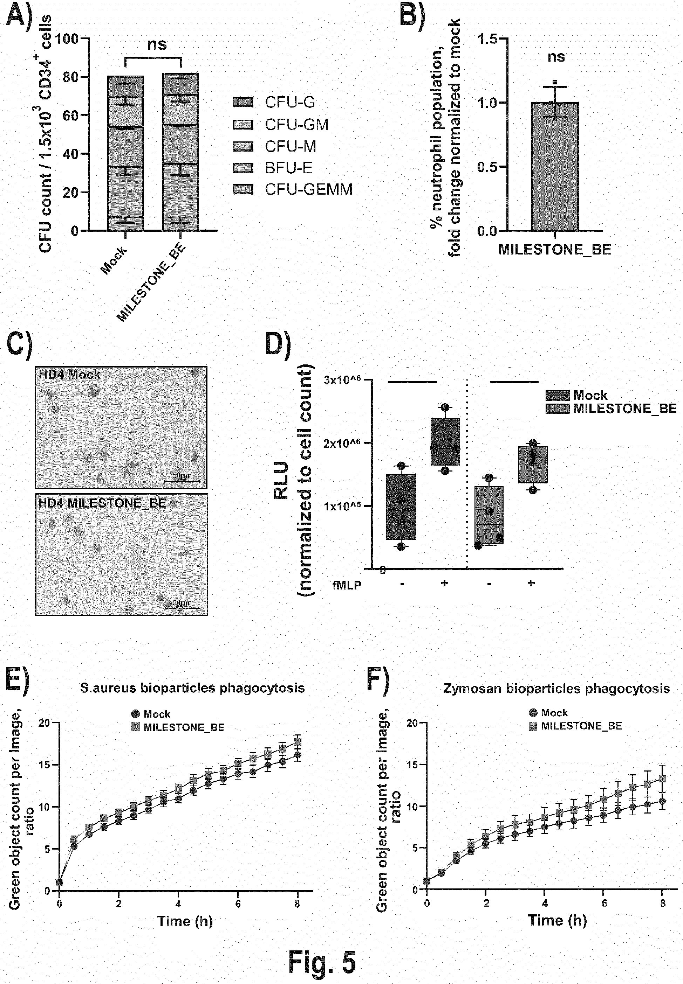

- Fig. 5: Analysis of *ELANE* promoter adenine base editing outcomes in healthy donors. A, CFU assay shows no significant changes in the average number of CFU colonies between the Mock and MILESTONE_BE cells. Data represent means ± SD from four biological replicates. One-way ANOVA with Dunnett's test, ns: not significant B, Liquid culture differentiation of healthy controls and MILESTONE_BE-edited HSPCs was performed to assess granulocytic differentiation. After 14 days of culture, FACS analysis revealed no significant differences in the CD16⁺CD66⁺CD45⁺ neutrophil population. Data represent means ± SD from four biological replicates. unpaired Student's t-tests, ns: not significant. C, Cytospin slides of Mock or MILESTONE_BE were stained with May-Grunwald - Giemsa and representative images are depicted, scale bar represents 50 µm. D, *In vitro* generated neutrophils on day 14 of liquid culture differentiation harvested from the Mock or MILESTONE_BE cells and were stimulated with formylmethionine-leucyl-phenylalanine (fMLP) for 30 min. The level of hydrogen peroxide (H₂O₂), a reactive oxygen species (ROS), was measured using GloMax Multidetection System plate reader. Data represent means ± SD from four biological replicates. unpaired Student's t-tests, *P < 0.05, RLU: Relative Light Unit. E, F, Phagocytosis kinetics of pHrodo Green *S*. *aureus* (E) and pHrodo Green *Zymosan* (F) bioparticles by neutrophils derived from liquid culture differentiation on day 15 from the Mock and MILESTONE_BE cells, analyzed using the IncuCyte Zoom System. The ratio of green object counts to the initial time point is presented. Data represent means ± standard error of the mean (SEM) from three biological replicates.

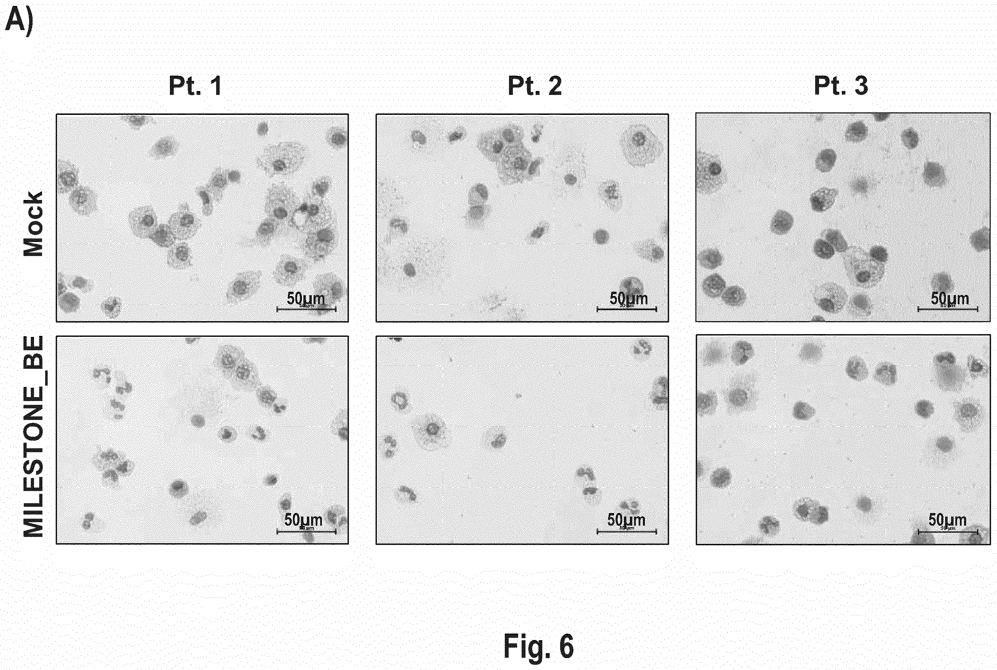

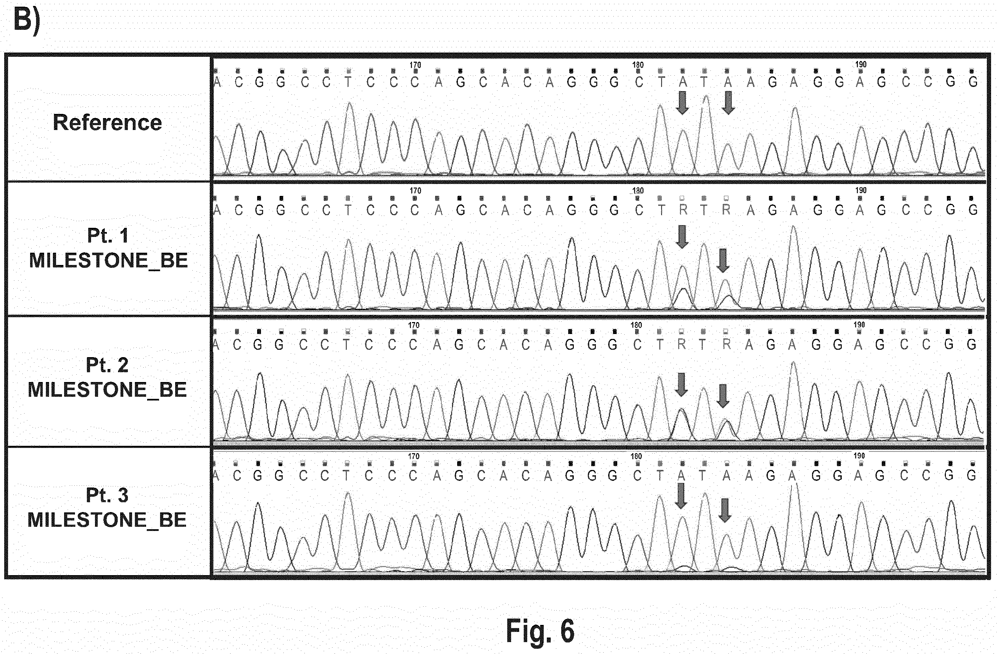

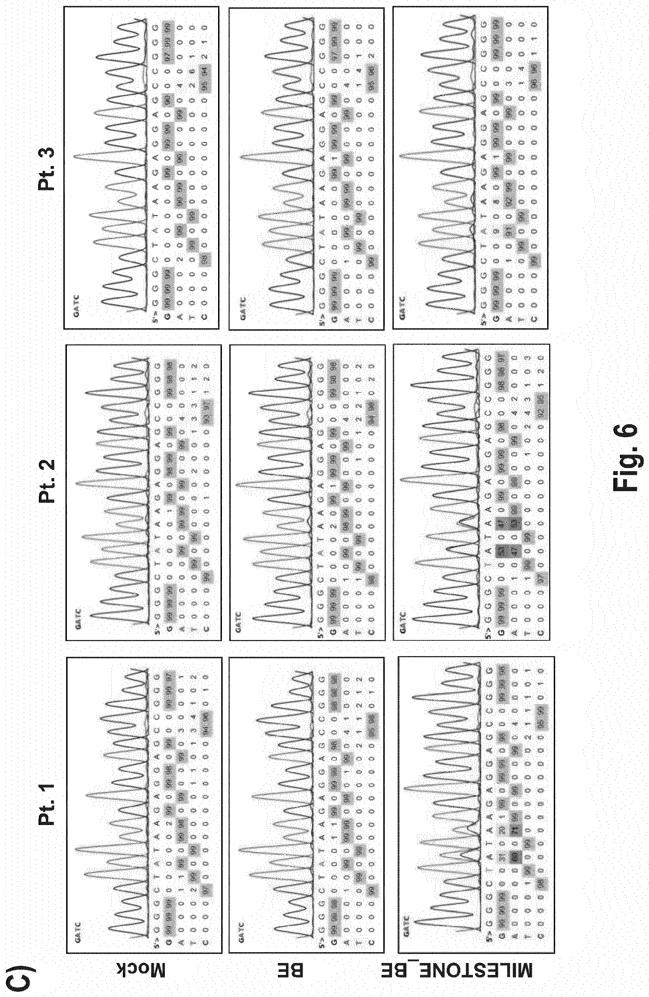

- Fig. 6: Analysis of MILESTONE_BE edited CN patients' HSPCs. A. Cytospin slides of Mock or MILESTONE_BE were stained with May-Grunwald - Giemsa and representative images are depicted. Scale bar is 50 µm. B. Representative Sanger sequencing traces of DNA isolated from CN patient-derived HSPCs 120 hours post-electroporated with Mock or MILESTONE_BE using Chromas Pro. The arrows indicate the specific target adenine base editing sites within the DNA sequence. These sites are the intended locations where the base editor enzyme will attempt to modify the adenine nucleotide to a guanine nucleotide. C. Representative Sanger sequencing traces of DNA isolated from patient-derived HSPCs 120 hours post-electroporation with Mock and MILESTONE_BE, analyzed using the online base editing analysis tool BEAT are shown. The calculated percentage of each base at each position is depicted.

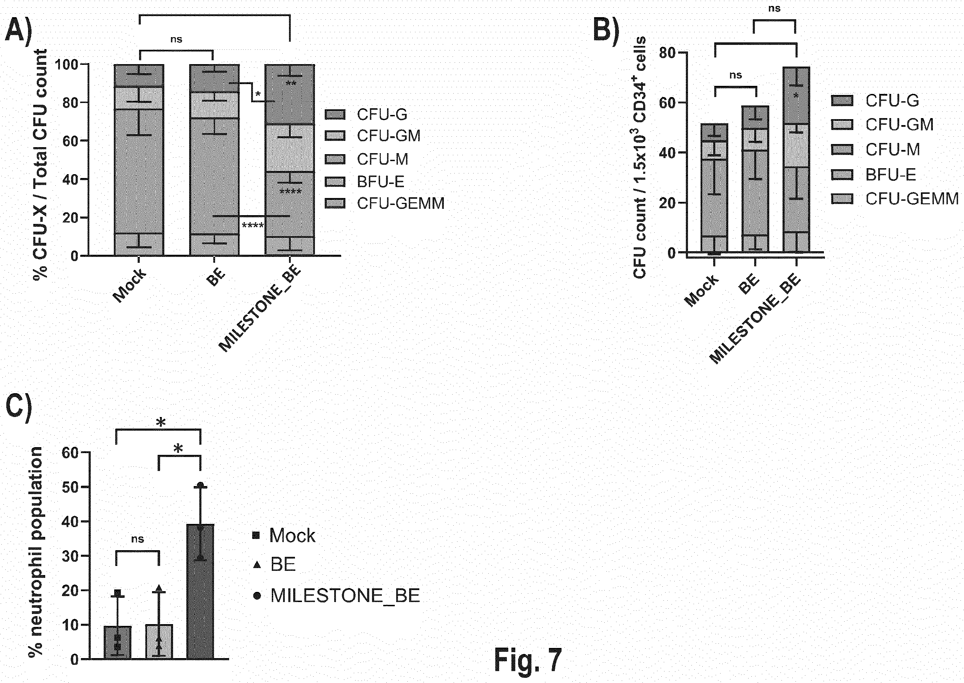

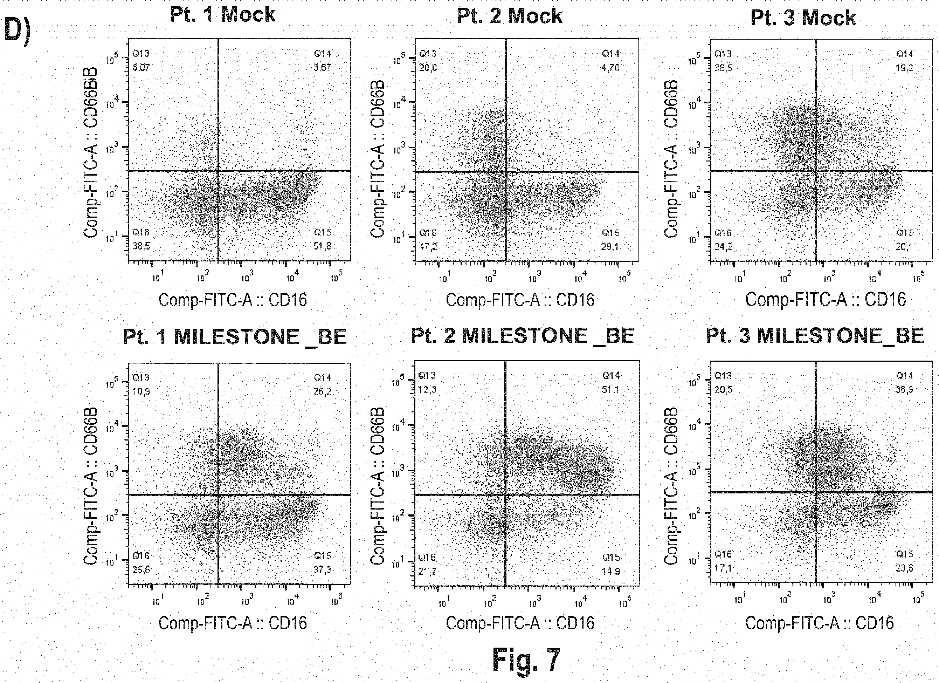

- Fig. 7: *ELANE* promoter base-editing restored granulocytic differentiation of congenital neutropenia patient HSPCs. A, CFU assay of the MILESTONE_BE cells showed an increase in the percentage of CFU-G colonies and a decrease in the percentage of CFU-M colonies, compared to the Mock control. Data represent means ± SD from three biological replicates. Two-way ANOVA with Tukey's test, **P < 0.01, ****P < 0.0001, ns: not significant. B, CFU assay of the MILESTONE_BE cells showed higher CFU-G colonies counts compared to the Mock. Data represent means ± SD from three biological replicates. Two-way ANOVA with Dunnett's test, *P < 0.05, ns: not significant. C, D, Liquid culture differentiation of congenital neutropenia patient's HSPCs showed an increased percentage of mature neutrophil population (CD45⁺CD66b⁺CD16⁺) derived from CD34⁺ cells of MILESTONE_BE compared to the Mock or ABE8.20-m mRNA electroporated (BE) controls. Data represent means ± SD from three biological replicates. One-way ANOVA with Tukey's test, *P<0.05, ns: not significant. D, Representative FACS images showing CD16⁺ CD66b⁺ mature neutrophils (black gate) in Mock or MILESTONE_BE groups are depicted.

### EXAMPLES

### 1. Material and methods

### Cell isolation and culture

Three severe congenital neutropenia patients harboring ELANE mutations (ELANE-CN) and four healthy donors were used in the study. Human CD34⁺ HSPC were isolated from bone marrow mononuclear cells of patients and cord blood of heathy donors by Ficoll gradient centrifugation and magnetic bead separation using human CD34⁺ progenitor cell isolation kit (Miltenyi Biotech, #130-046-703). CD34⁺ cells were cultured in a density of 2 x 10^5 cells/mL in StemSpan^{™} SFEM II hematopoietic stem cell expansion medium (StemCell Technologies, #09605) supplemented with 1 % penicillin/streptomycin, 1 % L-glutamine and a cytokine cocktail consisting of 20 ng/mL IL-3, 20 ng/mL IL-6, 20 ng/mL TPO, 50 ng/ml SCF and 50 ng/mL FLT-3L (all cytokines were purchased from R&D Systems).

Bone marrow and peripheral blood samples were collected from the patients in association with an annual follow-up recommended by the Severe Chronic Neutropenia International Registry (SCNIR). Informed written consent was obtained from all the study participants. The study was conducted according to Helsinki's declaration. Study approval was obtained from the Ethical Review Board of the Medical Faculty, University of Tübingen.

THP-1 cells were maintained in RPMI-1640 supplemented with 10% fetal bovine serum (FBS) (Gemini Bio Products, West Sacramento, CA, USA), 2 mM L-glutamine, and 1% penicillin/streptomycin (Thermo Fisher Scientific) at 37°C and 5% CO₂.

### Cloning and mRNA production

ABE8.20-m (Addgene plasmid #136300) was cloned into linearized pT7 vector for IVT (Addgene Plasmid 178113) with the use of NEBuilder^{®} HiFi DNA Assembly Cloning Kit (New England Biolabs, E5520S). The backbone of the cloned construct pT7-ABE8.20-m contains an inactive T7 promotor which prevents the *in vitro* transcription from the plasmid. ABE8.20-m was amplified (Takara Bio, PrimeSTAR^{®} Max DNA Polymerase, #R045B) using pT7-ABE8.20-m as a template and forward primer correcting T7 promoter sequence and reverse primer consisting of polyA tail. For the *in vitro* transcription, the PCR product was first purified using QIA quick PCR Purification Kit (QIAGEN, # 28106). According to the manufacturer's instructions, *in vitro* transcription was performed using the HiScribe^{™} T7 mRNA Kit with CleanCap^{®} Reagent AG (New England Biolabs, #E2080S) and Pseudo-UTP (tebu-bio, #040N-1019-1) instead of UTP. The mRNA was purified via LiCl precipitation following a quality (Agilent RNA 6000 Pico Kit, # 5067-1513) and quantity (Qubit RNA HS Assay Kit, # Q32852) determination. The produced mRNA was aliquoted and stored at -80°C upon use.

### CRISPR/Cas9 adenine base editing mediated ELANE promoter KO in HSPCs

Nucleofection was carried out using the Amaxa nucleofection system (P3 primary kit, #V4XP-3024) according to the manufacturer's instructions. For 3 x 10⁵ human CD34⁺ HSPCs, 300 pmol sgRNA and 3 ug ABE8.20-m mRNA was used for each nucleofection.

### Assessing genome editing efficiency

Gene editing efficiency was assessed either by Sanger sequencing of the targeted promoter region or qRT-PCR.

### Liquid culture differentiation of CD34⁺ cells

CD34⁺ cells were seeded in a density of 2 x 10⁵ cells/ml. Cells were incubated for 7 days in RPMI 1640 GlutaMAX supplemented with 10 % FBS, 1 % penicillin/streptomycin, 5 ng/ml SCF, 5 ng/ml IL-3, 5 ng/ml GM-CSF and 1 ng/ml G-CSF. Medium was exchanged every second day. On day 7, cells were plated in RPMI 1640 GlutaMAX supplemented with 10 % FBS, 1 % penicillin/streptomycin and 1 ng/ml G-CSF. On day 14, cells were analyzed by flow cytometry using following mouse anti-human antibodies: CD34 (BD, #348811), CD33 (BioLegend, #303416), CD45 (BioLegend, #304036), CD11b (BD, #557754), CD15 (BD, #555402), CD16 (BD, #561248), CD66b (Biolegend, #305104). Morphology of the cells was investigated on cytospin slides after May-Grunwald-Giemsa staining.

### 2. Results

The inventors have developed a gene editing approach for *ELANE*-CN through inhibition of the *ELANE* mRNA expression by converting two adenine nucleotides to guanine nucleotides in the *ELANE* promoter TATA box (TATA > TGTG) using CRISPR/Cas9 adenine base editor (ABE) (Figure 1A). First, the inventors screened the base conversion effect of four sgRNAs (Table 1). The sgRNAs were selected based on their position on the *ELANE* core promoter (Table 2), -2, -5, -29, and -81 nucleotide upstream of transcription start site, along with ABE mRNA (ABE8.20-m) on the *ELANE* promoter.

**Table 1: List of sgRHA sequences**

| sgRNA | Sequence |
|---|---|
| sgRNA -2 | 5'- GGGCTATAAGAGGAGCCGGG -3' (SEQ ID NO: 1) |
| sgRNA -5 | 5'- ACAGGGCTATAAGAGGAGCC -3' (SEQ ID NO: 2) |
| sgRNA -29 | 5'- TCCCAGCACAGGGCTATAAG -3' (SEQ ID NO: 3) |
| sgRNA -81 | 5'- TCATCAACGCCCTGTGCCAG -3' (SEQ ID NO: 4) |

**Table 2: ELANE core promoter sequence**

| Essential *ELANE* promoter region |
|---|
| 5'- TTCATCAACGCCCTGTGCCAGGGGAGAGGAAGTGGAGGGCGCTGGCCGGCCGTGG GGCAATGCAACGGCCTCCCAGCACAGGGCTATAAGAGGAGCCGGGCGGGCACGGA G -3' (SEQ ID NO: 5) |

The inventors found that modifying TATA box using mRNA ABE in the THP-1 reporter cell line, constitutively expressing high levels of *ELANE,* led to markedly reduced *ELANE* mRNA expression levels up to 70% five days post-electroporation, as assessed by measuring the luminescence levels of endogenously tagged NE protein (Figure 1B).

Both sgRNAs -2 and -5 were targeting the TATA box and showed the same editing and functional efficiency. The -5 sgRNA converted 3 adenines to guanines (A3: >15%, A2: >90%, A1: >90%), and the -2 sgRNA converted 2 adenines to guanines (A2: >90% and A1: >90%) (Figure 2A-B). Due to its uniform effect, the -2 sgRNA was selected for further development. qRT-PCR for *ELANE* mRNA expression also confirmed the screening results for the -2 sgRNA along with ABE8.20-m mRNA (Figure 1C). The on-target editing efficiency was 98%, as assessed by analysis of Sanger sequences traces five days post-electroporation (Figure 1D; Figure 2A-D).

ABE editing efficiency of *ELANE*'s TATA box in healthy donor hematopoietic stem and progenitor cells (HSPCs) (n = 4) was 83 to 97% (Figures 3 and 4) and granulocytic differentiation of these cells after ABE editing was unaffected, as assessed by colony-forming unit (CFU) assay and liquid culture granulocytic differentiation, compared to the Mock electroporated group (Figure 5A-C).

Furthermore, the *in vitro* generated ABE-edited neutrophils were functional, being able to produce reactive oxygen species (ROS) upon activation with N-Formyl-Met-Leu-Phe (fMLP) and perform phagocytosis of pHrodo green *Staphylococcus aureus* bioparticles and pHrodo Green Zymosan Bioparticles (Figure 5D-F).

Next, HSPCs from three *ELANE*-CN patients were also edited using the proposed strategy. The gene editing efficiency ranged from 8% to 53% (Figure 6C). ABE-*ELANE*-edited HSPCs from *ELANE*-CN patients exhibited a significant increase in neutrophilic differentiation, compared to the Mock electroporated group (Figure 7C, D; Figure 6).

To comprehensively evaluate the safety profile of *ELANE*-ABE editing of HSPCs, the inventors employ CAST-Seq for quantitative evaluation of chromosomal rearrangements, rhAmpSeq for accurate quantification of CRISPR-Cas on- and off-target edits, optical genome mapping to accurately detect all classes of structural variants (SVs) and RNA-seq to identify transcriptome-wide off-target RNA editing induced by *ELANE-*ABE editing. Preliminary data (not shown here) are very promising.

In conclusion, the findings of the inventors demonstrate the potential of *ex vivo* CRISPR/Cas9 adenine base editor (ABE) as a curative therapy of congenital diseases, e.g. for *ELANE*-CN patients. This innovative approach offers a double-strand break-free strategy that precisely targets the regulatory TATA box of the promoter of the gene of interest without altering the coding sequence of the gene and restoring granulopoiesis by reducing the expression level of gene of interest. The inventors believe that this approach has the potential to find a wide range of translational and clinical applications in the future.

## Claims

1. A composition for the targeted inhibition of the expression of a gene of interest in a biological cell, comprising:
- a protein comprising adenine deaminase activity or a nucleic acid molecule encoding said protein, and a
- a single guide RNA (sgRNA) configured to hybridize to a TATA box of a promoter controlling the expression of the gene of interest.

2. The composition of claim 1, wherein the protein is a CRISPR/Cas9 adenine base editor (ABE).

3. The composition of claim 1 or 2, wherein said gene of interest is a non-essential gene, preferably said non-essential gene is associated with a congenital disease, preferably if present in mutated form, further preferably in a gain-of-function mutated form.

4. The composition of claim 3, wherein said congenital disease is selected from the group consisting of: congenital neutropenia (CN), cyclic neutropenia (CyN), achondroplasia, Noonan syndrome, autosomal dominant forms of epilepsy, CAMK2-associated developmental disorders, androgen insensitivity.

5. The composition of any of the preceding claims, wherein the gene of interest is selected from the group consisting of: neutrophil elastase (*ELANE*), proprotein convertase subtilisin/kexin type 9 (*PCSK9*), fibroblast growth factor receptor 3 (FGFR3), protein tyrosine phosphatase non-receptor type 11 (*PTPN11*), son of sevenless homolog 1 (*SOS1*), Ca⁺⁺/calmodulin-dependent protein kinase II (*CAMK2*), androgen receptor (*AR*).

6. The composition of any of the preceding claims, wherein said biological cell is a hematopoietic cell, preferably a hematopoietic stem cell, further preferably primary human CD34⁺ hematopoietic stem cells, and highly preferably primary human CD34⁺ hematopoietic stem cells from patients with autosomal dominant gain-of-function mutations in *ELANE.*

7. The composition of any of the preceding claims, wherein said sgRNA comprises a nucleotide sequence selected from the group consisting of: SEQ ID NO: 1 (sgRNA -2), SEQ ID NO: 2 (sgRNA -5), SEQ ID NO: 3 (sgRNA -29), and SEQ ID NO: 4 (sgRNA -81).

8. The composition of any of the preceding claims, wherein the promoter comprises a nucleotide sequence of SEQ ID NO: 5.

9. The composition of any of the preceding claims, wherein said nucleic acid molecule encoding said protein comprising adenine deaminase activity is mRNA.

10. The composition of any of the claims 2-9, wherein said ABE is ABE8, preferably ABE8.20-m.

11. A method for the targeted inhibition of the expression of a gene of interest in a biological cell, comprising the following steps:
1) providing a biological cell comprising a gene of interest controlled by a promoter comprising a TATA box,
2) introducing into said biological cell a composition comprising:
- a protein comprising adenine deaminase activity or a nucleic acid molecule encoding said protein, and a
- a single guide RNA (sgRNA) configured to hybridize to said TATA box of said promoter controlling the expression of said gene of interest,
3) incubating said cell and said composition, thereby allowing the binding of the protein comprising adenine deaminase activity to the promoter and converting adenine nucleotides in the TATA box to guanine nucleotides, preferably two adenine nucleotides to guanine nucleotides (TATA -> TGTG).

12. The method of claim 11, wherein said composition is the composition of any of claims 1-10.

13. A preparation comprising a biological cell prepared *in vitro* by a method comprising the following steps:
1) providing a biological cell comprising a gene of interest controlled by a promoter comprising a TATA box,
2) introducing into said biological cell a composition comprising:
- a protein comprising adenine deaminase activity or a nucleic acid molecule encoding said protein, and a
- a single guide RNA (sgRNA) configured to hybridize to said TATA box of said promoter controlling the expression of said gene of interest,
3) incubating said cell and said composition, thereby allowing the binding of the protein comprising adenine deaminase activity to the promoter and converting adenine nucleotides in the TATA box to guanine nucleotides, preferably two adenine nucleotides to guanine nucleotides (TATA -> TGTG),
4) recovering said cell.

14. A kit for the targeted inhibition of the expression of a gene of interest in a biological cell, comprising the composition of any of claims 1-10 and instructions for delivering the composition to said biological cell so as to inhibit the expression of the gene.

15. A nucleic acid molecule, preferably an RNA molecule, comprising a nucleotide sequence which is selected from the group consisting of: SEQ ID NO: 1 (sgRNA -2), SEQ ID NO: 2 (sgRNA -5), SEQ ID NO: 3 (sgRNA -29), and SEQ ID NO: 4 (sgRNA -81).
